(19) Europäisches Patentamt
European Patent Office
Office européen des brevets

(11) EP 0 861 825 A1

# (12) DEMANDE DE BREVET EUROPEEN

(43) Date de publication:
**02.09.1998 Bulletin 1998/36**

(51) Int. Cl.[6]: **C07C 209/62**

(21) Numéro de dépôt: **98108235.7**

(22) Date de dépôt: **19.11.1993**

(84) Etats contractants désignés:
**AT BE CH DE DK ES FR GB GR IE IT LI LU NL PT SE**

(30) Priorité: **25.11.1992 FR 9214157**
**25.11.1992 FR 9214160**
**25.11.1992 FR 9214155**

(62) Numéro(s) de document de la (des) demande(s) initiale(s) en application de l'article 76 CBE:
**93402808.5 / 0 599 704**

(71) Demandeur: **RHODIA CHIMIE**
**92408 Courbevoie Cédex (FR)**

(72) Inventeur: **Ricca, Jean-Marc**
**75012 Paris (FR)**

(74) Mandataire:
**Ricalens, François et al**
**RHODIA CHIMIE**
**Direction de la Propriéte Industrielle**
**25, quai Paul Doumer**
**92408 Courbevoie Cédex (FR)**

Remarques:
Cette demande a été déposée le 06 - 05 - 1998 comme demande divisionnaire de la demande mentionnée sous le code INID 62.

## (54) Voie de synthése vers des anilines désactivées

(57) La présente invention a pour objet la synthèse d'anilines désactivées.

Ce procédé se définit en ce qu'il comporte au moins une étape consistant à faire réagir un composé de formule (I) :

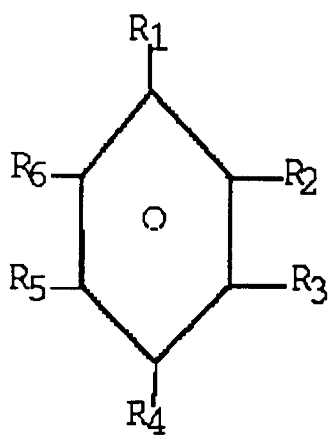

(I)

avec :

-$R_1$ choisi parmi les groupes dont les anions ($R_1^-$) constituent d'excellents groupes partants, avantageusement parmi les atomes de brome ou de chlore, de préférence ce demier ; sont considérés comme bon groupes partant, ceux dont l'acide associé à l'anion présentent une valeur de pKa au plus égale à 1, avantageusement à 0, de préférence à -1 ;

-$R_2$ choisi parmi l'hydrogène, les chaînes hydrocarbonées, les halogènes, les groupes électro-attracteurs (GEA) avantageusement par effet inducteur mais non mésomère ;

EP 0 861 825 A1

Printed by Xerox (UK) Business Services
2.16.3/3.4

(Cont. page suivante)

- $R_3$ est indifférent mais il est avantageux qu'il soit choisi parmi l'hydrogène et les groupes au moins aussi attracteurs par effet inductif que les groupes alcoyles ;

- $R_4$ est choisi parmi l'hydrogène, les chaînes hydrocarbonées les halogènes et les groupes électro-attracteurs avantageusement par effet inducteur mais non mésomère ;

- $R_5$ est indifférent mais il est avantageux qu'il soit choisi parmi l'hydrogène, les groupes au moins aussi attracteurs par effet inductif que les groupes alcoyles ;

- $R_6$ choisi parmi les chaînes hydrocarbonées les halogènes les groupes électroattracteurs, avantageusement par effet inducteur mais non mésomère ; avec la condition que au moins un des groupes $R_1$, $R_6$ et de préférence $R_4$ est électro attracteur par effet inductif;

  $R_2$ et $R_3$ et/ou $R_5$ et $R_6$ pouvant former au moins un cycle, éventuellement aromatique, le cas échéant substitué, sur un dialcoylamide en présence d'une base et à une température comprise entre 150 et 300°C de préférence entre 180°C et 250°C (dans la présente description, sauf mention contraire, les zéros de position ne sont pas significatifs).

Application à la synthèse organique.

## Description

La présente invention a pour objet la synthèse d'anilines désactivées.

Elle concerne plus particulièrement un procédé comportant une étape de dialcoylamination au moyen d'un amide.

La synthèse des anilines se fait en général au moyen des dérivés nitrés que l'on hydrogène.

Toutefois, cette technique est soumise à de nombreuses contraintes et n'est pas toujours facile à appliquer lorsque des produits sont sensibles à l'hydrogénolyse ou lorsque elle donne lieu à de nombreuses réactions parasites.

En outre, le positionnement de la fonction nitrée n'est pas toujours aisé et certaines synthèses nécessitent de nombreuses étapes.

La présente invention vise à apporter une nouvelle voie de synthèse utilisable pour faire des anilines substituées par un groupe électroattracteur (GEA), notamment en para.

Ce but, ainsi que d'autres qui apparaîtront par la suite sont atteints au moyen d'un procédé comportant une étape consistant à faire réagir un composé de formule (I)

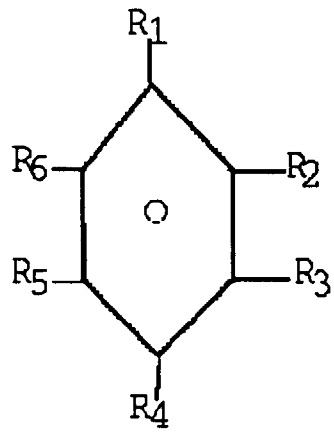

(I)

avec :

- $-R_1$ choisi parmi les groupes dont les anions ($R_1^-$) constituent d'excellents groupes partants, avantageusement parmi les atomes de brome ou de chlore, de préférence ce dernier ; sont considérés comme bon groupes partant, ceux dont l'acide associé à l'anion présentent une valeur de pKa au plus égale à 1, avantageusement à 0, de préférence à -1.
- $-R_2$ choisi parmi l'hydrogène, les chaînes hydrocarbonées, les halogènes, les groupes électro-attracteurs (GEA) avantageusement par effet inducteur mais non mésomère ;
- $-R_3$ est indifférent mais il est avantageux qu'il soit choisi parmi l'hydrogène et les groupes au moins aussi attracteurs par effet inductif que les groupes alcoyles ;
- $-R_4$ est choisi parmi l'hydrogène, les chaînes hydrocarbonées les halogènes et les groupes électro-attracteurs par effet inducteur mais non mésomère ;
- $-R_5$ est indifférent mais il est avantageux qu' il soit choisi parmi l'hydrogène, les groupes au moins aussi attracteurs par effet inductif que les groupes alcoyles ;
- $-R_6$ choisi parmi les chaînes hydrocarbonées les halogènes les groupes électroattracteurs, avantageusement par effet inducteur mais non mésomère ; avec la condition que au moins un des groupes $R_1$, $R_6$ et de préférence $R_4$ est électro-attracteur par effet inductif ;

$R_2$ et $R_3$ et/ou $R_5$ et $R_6$ pouvant former au moins un cycle, éventuellement aromatique, le cas échéant substitué ; sur un dialcoylamide en présence d'une base et à une température comprise entre 150 et 300°C de préférence entre 180°C et 250°C (dans la présente description, sauf mention contraire, les zéros de position ne sont pas significatifs).

Un des éléments de surprise vient du fait que l'on ait pu trouver des conditions où la réaction fonctionne avec moins de deux groupes nitro sur un même noyau et même sans.

Avantageusement, soit du fait de leur nature soit du fait de la disposition des groupes Électroattracteurs, les anions ($R_1^-$) constituent de meilleurs groupes partants que les anions issus des radicaux $R_2$ à $R_6$.

Avantageusement, le(s) groupe(s) électroattracteur(s) (GEA) est (sont) choisi(s) parmi les groupes pseudo-halogènes et alcoxycarbonyles, halogène léger (Fluor, chlore, brome), le plus souvent chlore et avantageusement perhalogénoalcoyle, et plus avantageusement trihalogénométhyle, de préférence perfluoroalcoyle.
Les radicaux $R_3$ et $R_5$ sont avantageusement peu encombrants et de préférence des hydrogènes, des pseudo-halogènes ou des halogènes, avantageusement fluor ou chlore à condition qu'ils soient moins bon groupe partant que $R_1$.
On entend par pseudohalogène ceux des radicaux susceptibles de constituer des groupes partants anioniques dont l'acide associé à l'anion présentent une valeur de pKa au plus égale à 1, avantageusement à 0, de préférence à -1.
On entend par perhalogénoalcoyle les groupes répondant à la formule

$$R - (CX_2)_{n-} \qquad \text{(III)}$$

→ où n est au moins égale à 1 et lorsque R n'est pas électroattracteur de préférence égal à 2
→ où les X sont choisis indépendamments parmis les halogènes de préférence parmi le fluor et le chlore,
→ où Y est un groupe partant de préférence le fluor, le chlore, le brome ou l'iode,
→ où R est un reste hydrocarboné, alcoyle ou aryle, de C1 à C10, avantageusement de préférence de C1 à C6 de préférence de C1 à C4, de préférence un électroattracteur, avantageusement un X (halogéne de préférence chlore ou fluor).

Les X sont le plus souvent identiques.
n est en général au plus égal à 10 et le plus souvent, notamment lorsque le perfluoroalcoyle est en position ortho, inférieur à 6, de préférence à 4.
Le solvant peut être tout solvant non-décomposable dans les conditions de l'expérience et inerte vis à vis des réactifs, avantageusement polaire (c'est-à-dire dont la constante diélectrique $\varepsilon$ est au moins égal à 30).
Lorsqu'il est protique, et a fortiori lorsqu'il ne l'est pas, il présente un pKa avantageusement au moins égal à celui de l'eau, à savoir 14.
Il va de soi que par solvant on entend tout mélange ou tout produit pur, susceptible d'être utilisé comme solvant.
Toutefois, on préfère travailler en utilisant comme seul solvant soit un excès de réactifs, soit un excès de substrat.
Parmi les bases utilisables, on préfère les bases anioniques y compris les carbonates. Les bases utilisées sont avantageusement les oxydes de métaux alcalins, des amidures alcalins, des hydroxydes et des alcoolates alcalins.
Pour obtenir de bon résultat, il est souhaitable que le pKa de l'acide conjugué de la base soit au moins égal à 9, avantageusement 10, de préférence à 12.
Il est à noter que le système tolère comme solvant les dérivés hydroxylés et notamment l'eau, surtout si l'on utilise du cuivre comme catalyseur.
Alors, avantageusement, la réaction est menée en présence de cuivre, ce dernier est de préférence introduit sous forme de sel cuivreux et en quantité comprise entre 0,1% et 10% en mole par rapport au substrat
Les quantités de réactifs et catalyseurs avantageusement mises en jeu sont les suivantes en prenant comme référence la quantité de substrat égal à 1 :

Base : de 0,5 à 5 équivalents-grammes, de préférence entre 2 et 4,
Amide : de 1 à 100 équivalents-grammes, de préférence de 5 à 25.

Les amides sont de préférence les dialcoylamides des acides carboxyliques légers (au plus 10 atomes de carbone).
Il est possible d'utiliser des diamides tel que les tétraméthylurées, on peut également utiliser la tetraméthylguanidine ; les meilleurs résultats sont obtenus lorsque les dialcoylamides sont des dialcoylformamides, les radicaux alcoyles greffés sur l'azote des amides étant avantageusement légers, de manières que le nombre d'atome de carbone n'excède 6, avantageusement 4, de préférence 3. Les dérivés méthylés sont les dérivés préférés.
La réaction a lieu en autoclave à la pression naturelle des réactifs dans les conditions expérimentales.
Quoique cela ne soit pas indispensable, il est préférable de travailler sous atmosphère inerte, par exemple sous azote ou sous argon.
L'invention présente un intérêt particulier pour obtenir des dérivés trifluorométhylés.
En effet, contre toute attente, les groupes perfluoroalcoyles, et notamment le groupe trifluorométhyle, sont stables dans les conditions opératoires de la présente invention.
Selon la présente invention, il a été ainsi possible de réaliser la synthèse de paratrifluorométhyl-N,N diméthylanilines en une seule étape à partir des dérivés halogénés, et notamment chlorés, correspondants.
Les résultats sont meilleurs lorsque l'on part d'un dérivé dihalogéné ou trihalogéné, de préférence dichloré ou trichloré, en position méta et para d'une fonction électro-attractrice perfluoroalcoyle, en général trifluorométhyle.
En d'autres termes la synthèse représente un intérêt particulier lorsque dans la formule (I) $R_1$est chlore ou brome de

préférence chlore ; $R_2$ est fluor, chlore ou brome de préférence chlore ; $R_3$ est hydrogène, fluor, chlore ou brome de préférence chlore ; $R_4$ est perfluoroalcoyle, en général trifluorométhyle.

Toutefois les composés obtenus par cette étape de la présente invention ne sont pas tous utilisables directement ; on peut même dire que l'aniline primaire, voire même secondaire, est plus intéressante que l'aniline tertiaire correspondante.

Or la technique usuelle pour déméthyler les anilines consiste à utiliser l'acide bromhydrique (HBr) aqueux qui risque de détruire les fonctions que l'on désire présentes sur le noyau conjointement à la fonction l'aniline. Comme fonction dont la fragilité est avérée, il convient de citer les fonctions halogénées, plus souvent perhalogénées en position benzylique, c'est à dire en $\alpha$ du noyau aromatique, en particulier la fonction perfluoroalcoyle, le plus souvent trifluorométhyle.
C'est pourquoi un des buts de la présent invention est de fournir un procédé de dé alcoylation des anilines notamment celles qui sont désactivées c'est à dire dont l'acide associé présente un pKa au plus égal à 2 de préférence à 1.

Un autre but de la présente invention est de fournir un procédé du type précédent qui n'abîme pas les fonction fragiles définies ci dessus, telle que la fonction perfluoroalcoyle, en général trifluorométhyle

Un autre but de la présente invention est de fournir un procédé du type précédent qui ne touche pas les substituants ni les positions en méta de l'aniline.

Ces buts, ainsi que d'autres qui apparaîtront par la suite sont atteints par un procédé pour N déalcoyler une aniline avantageusement désactivée, Mono- ou dialcoylée et il comporte l'étape suivante :

b) halogénation en elle même connue au moyen d'un agent d'halogénation de type radicalaire.

Cette halogénation est menée dans des conditions réputées radicalaires, avec, de préférence, une initiation (utilisation de radiation électromagnétique de longueur d'onde convenable, de peroxydes ou équivalents).

Elles présentent avantageusement de formule suivante (II) :

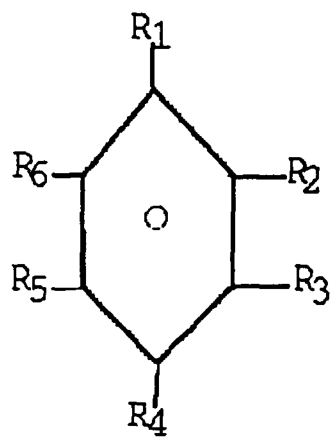

où $R_1$représente un radical mono- ou dialcoylamino- et

- -$R_2$ choisi parmi l'hydrogène, les chaînes hydrocarbonées les halogènes les groupes électro-attracteurs (GEA) par effet inducteur mais non mésomère ;
- -$R_3$ est indifférent mais il est avantageux qu'il soit choisi parmi l'hydrogène, les groupes au moins aussi attracteurs par effet inductif que les groupes alcoyles ;
- - $R_4$ est choisi parmi l'hydrogène, les chaînes hydrocarbonées les halogènes les groupes électro-attracteurs de préférence par effet inducteur mésomère ;
- -$R_5$ est indifférent mais il est avantageux que il soit choisi parmi l'hydrogène, les groupes au moins aussi attracteurs par effet inductif que les groupes alcoyles ;
- -$R_6$ choisi parmi les chaînes hydrocarbonées les halogènes les groupes électroattracteurs par effet inducteur mais non mésomère.

Dans un premier temps, s'il en reste de libre, les positions en ortho et para sont halogénées par l'agent d'halogénation.

Dans un deuxième temps, si on limite les quantités à la stoechiométrie (ou à un excès d'environ 10 % par rapport à la stoechiométrie pour une seule déalcoylation) mise en jeux de l'agent d'halogénation, un des groupes alcoyles et si

l'on ne limite pas les deux groupes alcoyles sont monohalogénés en position $\alpha$ pour donner des composés qu'il a été possible d'isoler et qui sont de structure :

$$Ar\text{-}N(\text{-}CH_{2\text{-}x}Z_x\text{-}R_1')(\text{-}CH_{2\text{-}y}Z_y\text{-}R_2') \qquad (III')$$

avec Z représentant l'Halogène apporté par l'agent Halogénant ;
$R_1$' étant un radical alcoyle d'au plus 4 carbones, de préférence un hydrogène et
$R_2$' étant un radical alcoyle d'au plus 4 carbones, de préférence un hydrogène.
$R_1$' et $R_2$' pouvant être reliés pour ne faire qu'un seul radical formant cycle avec l'azote.

On reviendra sur ce type de composé au cours de la description.

Cette déalcoylation est particulièrement surprenante quant à sa sélectivité puisque les positions méta ne sont pas touchées et que les carbones des chaînes alcoyles, situés en $\alpha$ de la fonction aniline ne sont pas touchés sensiblement plus d'une fois ce qui permet un économie de réactifs coûteux et réduit les quantités d'effluents, il est même possible de sélectivement mono déalcoyler l'aniline (lato sensu).

Au cours de l'étude qui a mené à la présente invention il a été montré que cette halogénation ne porte que sur les carbones des chaînes alcoyles liés directement à la fonction aniline et sur les positions en ortho et en para de la fonction aniline si elles sont libres.

En général, cette étape d'halogénation a lieu à une température comprise entre 0 et 100°C de préférence aux alentours entre 0°C et 50°C dans des solvants chlorés ou simplement polaires, lorsque l'on utilise des solvants dont le point d'ébullition est inférieur à 100°C, il est pratique de travailler au reflux, le cas échéant sous pression réduite pour amener la température de reflux dans la zone préférée.

En général le procédé comporte l'étape suivante :

c) hydrolyse du produit de l'étape b) d'halogénation. L'hydrolyse est connue en elle-même et est menée de préférence en présence d'absorbeur des acides dégagés.

L'halogénation peut introduire dans la molécule divers halogènes choisis parmi le chlore le brome ou l'iode.
En général pour des raisons économique on préfère le chlore.

Toutefois lorsque l'on désire apporter un halogène spécifique sur le noyau il convient d'utiliser un agent d'halogénation apportant cet halogène, il en va de même lorsque l'on désire éviter une perhalogénation des positions libres ortho et para par rapport à l'aniline : dans ce cas l'on halogène par un agent introduisant un halogène de rang supérieur à ceux existant dans le noyaux, puis on deshydrogénohalogène par de moyen connus en eux même pour être sélectif.
On choisit comme solvant de cette halogénation des solvants inertes dans les conditions de l'expérience dont on peut citer comme paradigmes, des composés tels que le tétrachlorure de carbone, le chlorobenzène, le dichlorobenzène et l'acétonitrile, le plus souvent, l'agent d'halogénation est l'halogène moléculaire ou atomique.

Outre les halogènes eux mêmes, on peut citer comme agent d'halogénation, les halogénures de thionyle ou de sulfuryle $SO_nCL_2$ (avec n égale à 1 ou 2), le pentachlorure de phosphore $PCL_5$ , le mélange $PCL_3/CL_2$ les hypochlorites d'alcoyles et les composés de type $X_20$ (avec X représentant un halogène ; sauf fluor et iode).
Lorsque ces réactifs ne sont pas naturellement des réactif radicalaire (comme c'est le cas pour les halogénures de thionyle ou de sulfuryle) sont utilisés en présence d'agent ou de conditions favorisant la formation de radicaux libre et notamment sous l'action de rayonnement actinique connu en eux même pour favoriser la formation des radicaux $Hal^\bullet$.

Le choix de la stoechiométrie permet de choisir entre une Halogénation du noyau, une monodéalcoylation ou une dialcoylation.
Dans le cas du brome, il convient de citer, lorsqu'ils sont stables et pas trop toxiques, les composés correspondants aux chlorés ci-dessus ainsi que les composés du type N Bromo Succinimide (NBS).

Dans le cas de l'iode le meilleur agent iodant reste l'iode moléculaire.
Ainsi les dérivés obtenus à partir de dichloro 1,2 trifluorométhyl 4 benzène ou de paratrifluorométhylchlorobenzène peuvent être transformés en trifluorométhyl 4 chloro 2 halogéno 6 aniline, par une halogénation en elle même connue, suivie d'une hydrolyse.

De manière inattendue les produits intermédiaires de formule

$$Ar\text{-}N(\text{-}CH_{2\text{-}x}Cl_x\text{-}R_1')(\text{-}CH_{2\text{-}y}Cl_y\text{-}R_2') \qquad (III)$$

où x = y = 1
où Ar représente le radical issu de la formule (I) par suppression du radical $R_1$ en laissant une valence libre et chloration des positions ortho et méta éventuellement laissées libres ;

sont suffisamment stables pour pouvoir être isolés. et constituer des précurseurs de nombreux dérivés chimiques.

En outre leur isolement du milieu réactionnel permet d'obtenir une meilleure pureté de l'aniline totalement ou partiellement déalcoylée, la séparation est très facilitée par le fait que les composés où y = 1 et x = 1 sont très majoritaires. Le procédé marche bien surtout pour les anilines mono- ou dialcoylées (lato sensu) dont l'acide conjugué présente un pKa au plus égal à 2, avantageusement à 1, de préférence à 0.
Au cours de l'étude qui a mené à la présente invention il a également été montré qu'il était possible de déalcoyler sélectivement les mono- et les di-alcoylanilines selon la présente invention sans toucher le radical aromatique initial.

Cette technique consiste à mettre en oeuvre un procédé qui comporte l'étape suivante :

b') faire réagir la dite aniline avec de l'ammoniac ou une amine, avantageusement au plus secondaire, sous forme libre ou d'un de ses sels, en présence d'une quantité qui peut être catalytique de sel(s) de pyridine, avantageusement de halohydrate(s) de pyridine.

Cette surprenante réaction présente l'avantage pour les dialcoylanilines de pouvoir être rendue sélective et d'être possible même quand les alcoylanilines sont secondaires.
Le terme "aniline" doit être pris dans son acception la plus large c'est à dire qu'il désigne toute amine liée directement à un noyau aromatique (de préférence à un seul) appauvri en électron, cet appauvrissement est mesuré par le pKa de l'acide associé à la fonction aniline que l'on désire déalcoyler.
Ainsi par aniline, il convient de comprendre non seulement l'aniline proprement dite, mais encore les composés chimiques provenant de la substitution de l'aniline y compris les anilines accolées à d'autre(s) noyau(x) tel(s) que par exemple les naphtylamines, l'appauvrissement du noyau en électron peut être du à toute cause connue tel que la présence de substituant(s) électroattracteur(s) de toute nature ou la présence dans un des cycles d'un hétéroatome (noyau à 6 chaînons).

Bien entendu par amine, il convient aussi de comprendre le mélange d'amines telles que définies ci-dessus ; l'amine peut être une pyridine.
Pyridine doit ici être entendue "lato sensu", c'est à dire comme toute molécule présentant un noyau pyridine dont l'atome d'azote ne possède pas de substituant.
Ainsi par pyridine, il convient de comprendre non seulement la pyridine proprement dite, mais encore les composés chimiques provenant de la substitution de la pyridine y compris les pyridines accolées à d'autre(s) noyau(x) tel(s) que par exemple la quinoléine. Il est préférable que les substituts de la pyridine proprement dite présente un point d'ébullition au plus égal à environ 200°C. Bien entendu par pyridine, il convient aussi de comprendre le mélange de pyridines telles que définies ci-dessus

Lorsque l'on désire déméthyler des diméthylanilines il est également possible de faire une mono ou une di-déalcoylation. Il y a mono déalcoylation lorsque l'acide correspondant au sel de pyridine et/ou au sel de l'amine est un acide relativement faible c'est à dire dont le pKa est au plus égal à environ 1, de préférence 3.

En général on utilise le chlorhydrate de pyridine, seul ou en présence de chlorhydrate d'amine.

Avantageusement, l'amine présente un pKa supérieur à celui de la pyridine de préférence d'au moins deux unités et plus préférentiellement de deux unité et demi.

Avantageusement, l'étape b') est menée à une température comprise entre 150 et 250°C, de préférence entre 180 et 220°C. Il convient de travailler avec un excès d'amine, de préférence avec un excès de chlorhydrate de pyridine, entre 1 et 50 équivalent, de préférence entre 2 et 10 (par rapport à la stoechiométrie de la déméthylation).

Un des aspects les plus surprenants et les plus intéressants de cette technique est que l'on ne constate aucune ammonolyse même dans le cas où l'aniline possède une fonction perfluoroalcoyle, en général trifluorométhyle.

La réaction est plus aisée lorsque le pKa de l'acide associé à l'aniline est au plus égal à 5, avantageusement à 2, de préférence à 0.

Les exemples, non limitatifs, suivants illustrent l'invention.

<u>**Exemples types**</u>

$$CF_3\text{-}\Phi\text{--}C \rightarrow CF_3\text{-}F\text{--}N<$$

Dans un autoclave chemisé Téflon (de 100ml), on introduit dans l'ordre du p.chloro trifluorométhylbenzéne (1,29 g, 7,15 millimoles) du DMF (4g) et de l'amidure de sodium (0,56g, 14,3mol).

L'autoclave est fermé et on chauffe sous agitation à 180°C pendant 24 h. Après traitement du milieu réactionnel, on obtient le para-trifluorométhyl N,N-diméthylaniline. (TT 74%, RT 77%).

* Influence du solvant :

$CF_3$-Φ--X→ $CF_3$-Φ--N<

X= Br ou de préférence Cl
Conditions : $CF_3$-Φ--CE 0,322 g (1,79 mmol)
$NANH_2$ 0,14 g 53,6 mmol)
DMF 4 g
autoclave chemisé Téflon (100 ml)

| **Solvants** | **Θ° (°c)** | **t(H)** | **RR** | **TT** | **RT** |
|---|---|---|---|---|---|
| **Exemple N° 1** DMF | 210 | 24 | 57 | 82 | 70 |
| **Exemple N° 2** Diméthyl acétamide | 220 | 24 | 30 | 76 | 37 |
| **Exemple N° 3** Tétraméthyl urée | 200 | 24 | 32 | 65 | 50 |
| **Exemple N° 4** Tétraméthyl guanidine | 21 | 24 | 30 | 96 | 32 |

* Plage de température: 150 - 230°C avec le DMF.

*

Cl NaOH Cl
$CF_3$ --- Φ- Cl ------------------> $CF_3$ --- Φ---- N <
DMF

Dans un autoclave d'1 litre en Hastelloy, on charge du dichloro- 3,4 trifluorométhylbenzéne (47,1 g , 0,212 mole), du DMF (200 g) et de la soude en pastilles (25,8 g, 0,645 mole).++

On ferme l'autoclave et on chauffe sous agitation à 210°C pendant 3 heures La pression atteint 9 bar.

Après traitement du milieu réactionnel, on obtient la trifluorométhyl-4 chloro-2 N,N-diméthylaniline (TT 92%) (RT 93%).

| ***Paramètres** | **Exemple N° 5** | **Exemple N° 6** | **Exemple N° 7** | **Exemple N° 8** | **Exemple N° 9** |
|---|---|---|---|---|---|
| Dichloro 3,4 trifluorométhyl-benzéne | 0,215 | 0,225 | 0,225 | 0,222 | 0,224 |
| (mmol) | 1 | 1 | 1 | 1 | 1 |
| DMF (g) | 4 | 4 | 4 | 4 | 4 |
| éq$^{ts}$(équivalents) | 55 | 55 | 55 | 55 | 55 |
| NaOH (g) | ------ | ------- | 0,044 | 0,048 | ------ |
| éq$^{ts}$ | ------ | ------- | 1,1 | 1,2 | ------ |
| $H_2O$ (g) | 0,210 | 0,211 | ------ | ------ | ------ |
| éq$^{ts}$(équivalents) | 12 | 12 | ------ | ------ | ------ |
| NaOH 37% (g) | ------ | ------- | ------ | ------ | 0,202 |
| éq$^{ts}$ (équivalents) | ------ | ------- | ------ | ------ | 1,8 NaOH 7 H2O |

(suite)

| *Paramètres | Exemple N° 5 | Exemple N° 6 | Exemple N° 7 | Exemple N° 8 | Exemple N° 9 |
|---|---|---|---|---|---|
| θ(=°C) | 195 | 195 | 195 | 195 | 195 |
| T (heures) | 20 | 20 | 20 | 20 | 20 |
| TT (%) | 45 | 75 | 59 | 78 | 91 |
| RT (%) | 87 | 86 | 71 | 67 | 64 |

## Exemple N° 10

Du chlorure de sulfuryle (9,5g, 70mmol) est ajouté à solution de chloro-2 trifluorométhyl-4 N,N-diméthyl aniline (14,25g, 63,8mmol) dans du tétrachlorure de carbone (90ml).

l'essai a été mené en présence d'une forte lampe et en l'absence de lumière.

Après chauffage à 70°C pendant 2 heures, la solution est traitée par un excès de soude à 50% dans l'eau.

L'analyse HPLC de la phase organique montre les résultats suivants :

| Conditions | dichloro-2,6 trifluorométhyl-4 N,N- diméthyl aniline | dichloro-2,6 trifluorométhyl-4 N-méthyl aniline |
|---|---|---|
| En présence de lampe | 5-10% | 85 % |
| A la lumière du jour | 68 % | 17 % |
| Dans l'obscurité | 90 % | 0 % |

## Exemple N° 11

Un mélange de dichloro-2,6 trifluorométhyl-4 N,N-diméthyl aniline (4,5g, 17,4mmol) et de dichloro-2,6 trifluorométhyl-4 N-méthyl aniline (1,68g, 6,9mmol) en solution dans du tétrachlorure de carbone (60ml) est traité par du chlorure de sulfuryle (5,7g, 42,2mmol).

Le mélange réactionnel refroidi à 10°C est illuminé par l'intermédiaire d'une lampe UV pendant 6 heures, puis traité par un excès de soude à 50% dans l'eau.

L'analyse CLHP de la phase organique montre la présence de dichloro-2,6 trifluorométhyl-4 aniline (70% molaire).

## Exemple N° 12

De la dichlor-2,6 trilfluorométhyl-4 N-méthyl aniline (1,318g, 5,4mmol) en solution dans du tétrachlorure de carbone (40ml) est traitée par du chlorure de sulfuryle (4,5g, 33mmol). Le mélange réactionnel refroidi à 10°C est illuminé par l'intermédiaire d une lampe pendant une heure, puis traité par un excès de soude à 50% dans l'eau. L'analyse CLHP de la phase organique montre la présence de la dichloro-2,6 trifluorométhyl-4 aniline (83% molaire).

## Exemple N° 13

Une quantité théorique de chlore gazeux est introduite dans une solution de dichloro-2,6 trifluorométhyl-4 N,N-diméthyl aniline (3g, 11,6mmol) en solution dans du tétrachlorure de carbone (60ml).

Pendant l'introduction de chlore, le mélange réactionnel est refroidi à 10°C et illuminé par l'intermédiaire d'une lampe UV. L'analyse CPHL de la phase organique après traitement, après 1 heure montre une conversion de 92% et un rendement en dichloro-2,6 trifluorométhyl-4 aniline de 64%.

## Exemple N° 14

Une quantité théorique de chlore gazeux est introduite dans une solution de dichloro-2,6 trifluorométhyl-4 N,N-diméthyl aniline (2,25g, 8,7mmol) en solution dans du dichloro-1,2 benzène (65ml).

Pendant l'introduction de chlore, le mélange réactionnel est refroidi à 10°C et illuminé par l'intermédiaire d'une lampe

UV.
L'analyse CLHP de la phase organique après traitement au bout de 50 minutes montre une conversion de 99% et un rendement en dichloro-2,6 trifluorométhyl-4 aniline de 92%.

**Exemple N° 15**

Une quantité théorique de chlore gazeux est introduite dans une solution refroidie à 10°C de dichloro-2,6 trifluorométhyl-4 aniline (8,45mmol) dans du dichloro-1,2 benzène (65ml). Après illumination de la masse réactionnel par l'intermédiaire d'une lampe UV pendant 15 minutes, la réaction est poursuivie à l'abri de la lumière pendant 2 heures. L'analyse CLHP de la phase organique après traitement montre une conversion de 98% et un rendement en dicholoro-2,6 trifluorométhyl-4 aniline de 34%.

**Exemple N° 16**

Dans un autoclave chemisé Téflon (de 100ml), on introduit dans l'ordre du trichloro-1,2,3 benzène (0,963g), du DMF (4g) et de la soude (0,4g).
L'autoclave est fermé et on chauffe sous agitation à 220°C pendant 24 heures. La conversion est alors de 78%.

Après traitement du milieu réactionnel, 0,517g du mélange de dichloro N,N-diméthyl anilines est mis au contact de chlorhydrate de pyridine (5,70g).

Le mélange est chauffé à 185°C pendant 45 minutes.
L'analyse CLHP du mélange montre la présence de dichloro-2,3 aniline (95%) et de dichloro-2,6 aniline (5%).

**Exemple N° 17**

Dans un autoclave chemisé Téflon (de 100ml), on introduit dans l'ordre du trichloro-1,2,4 benzène (0,766g), du DMF (4g) et de la soude (0,4g).
L'autoclave est fermé et on chauffe sous agitation à 220°C pendant 24 heures.
La conversion est alors de 82% .

Après traitement du milieu réactionnel, 0,223 g du mélange de chloro N,N-deméthyl anilines est mis au contact de chlorhydrate de pyridine (3,2g).

Le mélange est chauffé à 185°C pendant 60 minutes.

L'analyse CLHP du mélange montre la présence de dichloro-2,4 aniline (96%) et de dichloro-3,4 aniline (4%).

**Exemple N° 18**

Dans un autoclave chemisé Téflon (de 100ml), on introduit dans l'ordre du tétrachloro-1,2,4,5 benzène (0,919g), du DMF (4g) et de la soude (0,4g).
L'autoclave est fermé et on chauffe sous agitation à 220°C pendant 24 heures.
La conversion est alors de 100%.

Après traitement du milieu réactionnel, 0,50g du mélange de trichloro N,N-diméthyl anilines est mis au contact de chlorhydrate de pyridine (5,8g).
Le mélange est chauffé à 190°C pendant 60 minutes.
L'analyse CLHP montre la présence de trichloro-2,4,5 aniline (100%).

**Exemple N° 19**

Dans un autoclave chemisé Téflon (de 100ml), on introduit dans l'ordre du dichloro-2,6 bromobenzène (0,992g), du DMF (4g) et de la soude (0,4g).
L'autoclave est fermé et on chauffe sous agitation à 220°C pendant 24 heures.
La conversion est alors de 89%.

Après traitement du milieu réactionnel, 0,43g du mélange de chloro N,N-diméthyl anilines est mis au contact de chlorhydrate de pyridine (5,2g).
Le mélange est chauffé à 190°C pendant 60 minutes.
L'analyse CLHP du mélange montre la présence de dichloro-2,6 aniline (43%) et de bromo-2 chloro-3 aniline (57%).

**Exemple N° 20**

Du chlorhydrate de pyridine (4,35g, 37,7mmol) est ajouté à du chloro-2,6 trifluorométhyl-4 N,N-diméthyl aniline

(0,86g, 3,3mmol).

Le mélange est chauffé à 185°C pendant 2 heures.
L'analyse CLHP montre alors la présence de dichloro-2,6 trifluorométhyl-4 aniline (76% molaire).

**Exemple N° 21 monodéméthylation**

Dans un autoclave chemisé Téflon (de 100ml), on introduit du dichlor-2,6 trifluorométhyl-4 N,N-diméthyl aniline (0,86g, 3,3mmol) et de l'acétate de pyridinium (5g). L'autoclave est fermé et on chauffe à 190°C pendant 2 heures.
L'analyse CLHP montre la présence de dichloro-2,6 trifluorométhyl-4 N-méthyl aniline (rdt 9% molaire).

**Exemple N° 22 (comparatif)**

Un mélange de dichloro-2,6 trifluorométhyl-4 N,N-diméthyl aniline (0,810g) et de chlorhydrate de diméthylamine (3,5g) est chauffé 1 heure à 180°C.
L'analyse CLHP du mélange indique l'absence de conversion.

**Exemple N° 23**

Un mélange de dichloro-2,6 trifluorométhyl-4 N,N-diméthylamine (0,745g), du chlorhydrate de pyridine (34mg) et du chlorhydrate de diméthylamine (3,24g) est chauffé 1 heure à 180°C.
L'analyse CLHP du mélange montre un rendement(20 ù) en dichloro-2,6 trifluorométhyl-4 aniline trois fois supérieur à la théorie si la pyridine était seule active.

**Exemple N° 24 (technique usuelle)**

Une solution de dichloro-2,6 trifluorométhyl-4 N,N-diméthyl aniline (1g) dans l'acide bromhydrique aqueux (47%, 5g) est portée au reflux pendant 5 heures.
L'analyse CLHP du mélange montre une conversion de 89% et un rendement en dichloro-2,6 trifluorométhyl-4 aniline de 55%.

**Exemple N° 25 comparatif**

L'objectif est de démontrer la nécessité du caractére radicalaire.

Dans un réacteur téflon de 100 ml, on introduit du chloro-2 nitro-6 N,N-diméthyl aniline (0,802 g, 4 mmol) en solution dans du chloroforme (10 ml). A cette solution, on ajoute une solution de brome (0,64 g, 4 mmol) dans du chloroforme (20 ml). Le mélange est agité à température ambiante pendant 4 heures. L'analyse du mélange réactionnel ne montre que la seule présence du produit de départ.

(Technique d'analyse : chromato gaz et spectro de masse).

**Exemple N° 26**

On cherche à démontrer la présence nécessaire d'une base.

Technique sans base

Dans un autoclave chemisé Teflon (100 ml), on introduit dans l'ordre du dichloro-3,4 trifluorométhyl-4 benzène (4,62 g, 21,4 mmol) et du diméthylformamide (22 g, 14 équivalents molaires).
L'autoclave est fermé et on chauffe sous agitation pendant 5 Heures à 200°C.
L'analyse du milieu réactionnel par chromatographie en pahse gazeuse indique une conversion de 3 % pour un rendement en diméthylanilines de 2 %.

Technique avec base

Dans un autoclave chemisé Tefon (100 ml), on introduit dans l'ordre du dichloro-3,4 trifluorométhyl-4 benzène (4,62 g, 21,4 mmol), du diméthylformamide (22 g, 14 équivalents molaires).
L'autoclave est fermé et on chauffe sous agitation pendant 5 heures à 200°C.
L'analyse du milieu réactionel par chromatographie en phase gazeuse indique une conversion de 95 % pour un rendement en chloro-2 trifluorométhyl-4 diméthylaniline de 80 %.

**Exemple N° 27 de régénération des pyridiniums et procédé de recyclage**

Le chlorydrate de pyridine peut être facilement obtenu par des techniques connus de l'homme de l'art comme par exemple traitement de lapuridine avec une quantité stoechiométrique d'une solution d'acide chlorhydrique à 36 % dans l'eau puis distillation azéotropique de l'eau avec du toluène.

**Procédé :**

De la dichloro-2,6 trifluorométhyl-4 diméthyl aniline (1,72 g, 6,6 mmol) est chauffée à 180°C 1 heure en présence de chlorhydrate de pyridine (8,7 g, 37,7 mmol). Un dosage de la masse réactionnelle indique une conversion de 100%. Le milieu réactionnel est alors refroidi à 140°C et on ajoute sous agitation de l'ortho-xylène (5ml). Au bout de 2 Minutes, l'agitation est arrêtée et on assiste à une séparation en deux couches. La phase supérieure est récupérée.
L'analyse par chromatographie en phase gazeuse montre la présence de dichloro-2,6 trifluorométhyl-4 aniline (1,47g, rendement 97 %).
La phase inférieure contenant un mélange de chlorydrates de méthylpyridinium et pyridinium est régénérée à 200°C par l'action de l'ammoniac. La pyridine récupérée par distillation peut être recyclée.

**Revendications**

1. Procédé de déalcoylation des anilines caractérisé par le fait qu'il comporte l'étape suivante ;

   b') faire réagir la dite aniline avec de l'ammoniac ou une amine, avantageusement au plus secondaire, sous forme libre ou d'un de ses sels, en présence d'une quantité qui peut être catalytique de sel(s) de pyridine, avantageusement de halohydrate(s) de pyridine.

2. Procédé selon la revendication 1, caractérisé par le fait que l'amine présente un pKa supérieur à celui de la pyridine de préférence d'au moins deux unités et plus préférentiellement de deux unités et demie

3. Procédé selon les revendications 1 et 2, caractérisé par le fait que l'étape b') est menée à une température comprise entre 150 et 250°C, de préférence entre 180 et 220°C

Office européen des brevets

# RAPPORT DE RECHERCHE EUROPEENNE

Numéro de la demande
EP 98 10 8235

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| A | D. LSAMANN ET AL: "Eine neue Methode zur Entalkylierung am Stickstoff, I. Mittel.: Alkylabspaltung bei N-Alkylcarbonsäure-Amiden un -Aniliden" BERICHTE DER DEUTSCHEN CHEMISCHEN GESELLSCHAFT., vol. 87, no. 9, 1954, WEINHEIM DE, pages 1294-1300, XP002068856 * page 1294 * * page 1300, ligne 26 - ligne 51 * | 1-3 | C07C209/62 |
| A | M. FIESER, L.F. FIESER: "Reagents for Organic Synthesis. Volume 3" , WILEY-INTERSCIENCE , NEW YORK XP002068857 * page 239 - page 240 * | 1-3 | |

| DOMAINES TECHNIQUES RECHERCHES (Int.Cl.6) |
|---|
| C07C |

Le présent rapport a été établi pour toutes les revendications

| Lieu de la recherche | Date d'achèvement de la recherche | Examinateur |
|---|---|---|
| LA HAYE | 22 juin 1998 | Sánchez García, J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)